# DEMANDE DE BREVET EUROPEEN

(11) **EP 2 543 315 A1**
(43) Date de publication de la demande: **09.01.2013**
(21) Numéro de dépôt: 12170815.0
(22) Date de dépôt: 05.06.2012
(51) Int. Cl.: A61B 5/083, A61B 5/087, A61M 16/00, G01N 33/497, G01F 1/34, G01L 7/00, G01L 13/00

(54) **Système de mesure de pression pour ventilateur médical**

(30) Priorité: 08.07.2011 FR 1156209
(71) Demandeur: Air Liquide Medical Systems, 92160 Antony (FR)
(72) Inventeur: Boulanger, Thierry, 37000 TOURS (FR)
(74) Mandataire: Pittis, Olivier

(57) **Abrégé**

L'invention porte sur un système de mesure de pression (1) comprenant un élément de conduit (2), un sous-ensemble de mesure (3) et un système de connexion/déconnexion permettant de solidariser mécaniquement le sous-ensemble de mesure (3) à l'élément de conduit (2) ou, à l'inverse, de les désolidariser l'un de l'autre. L'élément de conduit (2) comprend un élément résistif (23) pour créer une perte de charge du gaz traversant ledit élément de conduit (2). Des orifices de mesure (14, 15) traversent la paroi (11) de l'élément de conduit (2) de part et d'autre de l'élément résistif (23). Par ailleurs, le sous-ensemble de mesure (3) comprend deux capteurs de pression (33, 34) reliés aux orifices de mesure (14, 15) lorsque le sous-ensemble de mesure (3) est solidarisé à l'élément de conduit (2). Des membranes (24, 25 ; 31, 32) sont agencées au niveau des orifices de mesure (14, 15) de l'élément de conduit (2) ou des canaux de prise de pression (31a, 32a) des capteurs de pression (33, 34). Tout ou partie des membranes (24, 25 ; 31, 32) est en céramique. Les membranes (24, 25 ; 31, 32) comprennent une multitude de pores dont la taille est inférieure à 100 nm. Installation de ventilation d'un patient comprenant un ventilateur (5) relié fluidiquement à un circuit patient (6), et un système de mesure de pression (1) selon l'invention.

## Description

L'invention concerne un système de mesure de pression permettant une mesure proximale des pressions et débits gazeux dans le cadre d'une ventilation mécanique, invasive ou non invasive.

L'assistance respiratoire de patients par le biais de ventilateurs, que ce soit de manière temporaire en anesthésie à l'occasion d'une intervention chirurgicale ou plus longuement en unités de soins critiques, exige une maîtrise constante des paramètres de ventilation et, en premier lieu, des pressions délivrées par le ventilateur et des débits ou volumes administrés.

Afin de garantir cette maîtrise les dispositifs de ventilation comprennent généralement des capteurs spécifiques qui viennent mesurer la pression des voies aériennes du patient et le débit délivré au patient par le ventilateur et souvent expiré par celui-ci

Or, il est connu que la meilleure précision ne peut être obtenue qu'à l'aide de capteurs placés au plus près du patient, c'est-à-dire de manière proximale afin d'éviter les effets délétères des accessoires ou autres éléments placés entre le ventilateur et le patient, tels que circuits, filtres bactériologiques, humidificateurs...

En effet, de tels composants introduisent par exemple une résistance supplémentaire non maîtrisée qui peut, dans certaines applications, introduire un biais dans la pression réellement administrée au patient. Par ailleurs, le volume de ces accessoires introduit un effet de compliance (exprimée en ml/cm H₂O) qui piège en se distendant, une partie du volume supposé être administré au patient.

Des mécanismes de compensation existent sur la plupart des ventilateurs mais une mesure exempte de ces effets indésirables est à privilégier. Ceci est d'autant plus vrai en pédiatrie où les volumes administrés peuvent être excessivement faibles, c'est-à-dire de l'ordre de 5 ml, et rendent donc une mesure proximale essentielle.

Or, réaliser une mesure proximale des paramètres de ventilation n'est pas sans difficultés.

Ainsi, un inconvénient majeur réside dans le fait qu'un capteur situé à proximité du patient et non dans le ventilateur est exposé à un environnement plus sensible, c'est-à-dire potentiellement contaminé par des germes, telles des bactéries, et souvent chargé en humidité qui, en se condensant, peut affecter la mesure du capteur, voire l'endommager.

Un tel environnement exige donc pour le capteur qui est en contact avec le gaz inspiré et expiré par le patient, d'être entièrement désinfecté, voire stérilisé, d'un patient à l'autre pour éviter toute contamination croisée, ce qui est une contrainte excessivement difficile à satisfaire.

Le document US-A-2007/261498 décrit un système de mesure de pression comprenant un passage de gaz avec restriction interne et deux orifices de prise de pression situés de chaque côté de la restriction. Des éléments de filtres hydrophobes ou antimicrobiens sont logés dans les orifices de manière à arrêter notamment les microbes. Toutefois, ce document ne donne aucune autre précision quant aux filtres à utiliser. En outre, ces filtres ne garantissent une protection efficace que pendant un laps de temps limité, typiquement 24h et ne sont pas réutilisables, c'est-à-dire qu'ils ne supportent pas les cycles de nettoyage. Dans le cadre d'une utilisation prolongée sur un patient, par exemple en service de réanimation, de tels filtres doivent alors être régulièrement remplacés, ce qui ajoute une charge au personnel soignant et engendre des coûts supplémentaires.

Par ailleurs, le document EP-A-437055 propose, pour un système analogue à celui du document précédent, d'utiliser des filtres poreux en polyéthylène ayant une taille de pore importante, à savoir une taille de pore de 10 µm Or, ce type de filtre n'est pas entièrement satisfaisant car, d'une part, il ne permet pas d'arrêter des microorganismes de petite taille, tels certains virus par exemple, et, d'autre part, il ne résiste pas aux opérations de désinfection et surtout de stérilisation à chaud. En effet, un cycle courant de désinfection/stérilisation intègre un passage à l'autoclave où l'élément est soumis à une température d'environ 134°C pendant plusieurs minutes. Dès lors, les capteurs proximaux doivent donc être robustes ou « à usage unique », c'est-à-dire remplacé à chaque nouveau patient. Lorsque les filtres sont en un matériau non adapté, ils sont détériorés lors du chauffage de stérilisation.

En fait, à ce jour, il n'existe qu'un seul capteur proximal permettant de réaliser une mesure simultanée de la pression et du débit. Ce capteur repose sur le principe des pertes de charge, c'est-à-dire qu'une lamelle insérée dans le corps du capteur se déforme sous l'effet du débit administré au patient et génère à ses bornes un différentiel de pression. Plus le débit est grand, plus ce différentiel de pression augmente. Deux tubulures, généralement de plusieurs mètres, viennent alors réaliser la connexion entre le corps du capteur, et plus précisément de part et d'autre de la lamelle, et le ventilateur où un capteur de pression vient mesurer la perte de charge occasionnée par le débit circulant dans le corps du capteur et en déduire par une table de conversion une approximation du débit réel. Un capteur de pression additionnel vient également effectuer une mesure en aval de la lamelle qui représente alors la pression des voies aériennes du patient.

Or, il apparaît que de si longues tubulures sont à éviter car:
- elles surchargent l'environnement patient qui est déjà encombré,
- elles peuvent se « clamper », c'est-à-dire être obstruées, ce qui interdit alors toute mesure fiable du débit et de la pression,
- elles sont soumises à l'effet de condensation de l'air précédemment humidifié qui se refroidit, ce qui nécessite un recalage fréquent des capteurs du ventilateur, et
- elles interdisent, de par leur longueur et l'effet de filtre associé, l'utilisation d'un tel capteur dans le cadre de ventilations à haute fréquence, c'est-à-dire de quelques hertz.

Pour éviter l'usage de telles tubulures, on pourrait déporter le capteur de pression à proximité du patient. Toutefois, ceci n'est pas possible car un tel capteur ne supporte ni la condensation de l'humidité, ni les cycles de désinfection/stérilisation susmentionnés.

De là, bien que remplissant sa fonction primaire qui est de mesurer un débit et une pression, un tel capteur n'est pas idéal pour les raisons susmentionnées.

Si l'on ajoute à cela la nature même du capteur, typiquement à usage unique nécessitant donc le remplacement de plusieurs mètres de tubulures ainsi que du corps du capteur, il serait souhaitable de disposer d'une solution alternative, exempte des limitations exposées.

En d'autres termes, le problème qui se pose est de pouvoir disposer d'un capteur de pression ou de débit qui, tout en remplissant sa fonction de mesure proximale de pression ou débit, ne soit pas exposé aux agents contaminants et autres condensats de vapeur d'eau provenant du circuit patient, de sorte que ledit capteur conserve toute sa fonctionnalité et ne nécessite aucun entretien ou remplacement à chaque patient de la part du personnel médical, et qui soit stérilisable à haute température, c'est-à-dire typiquement à plus de 100°C, sans se détériorer de manière à pouvoir être réutilisé.

La solution est alors un système de mesure de pression comprenant un élément de conduit, un sous-ensemble de mesure et un système de connexion/déconnexion permettant de solidariser mécaniquement le sous-ensemble de mesure à l'élément de conduit ou, à l'inverse, de les désolidariser l'un de l'autre, et dans lequel :
- l' élément de conduit comprend :
   - une paroi périphérique tubulaire délimitant un passage interne de gaz reliant fluidiquement un orifice d'entrée à un orifice de sortie,
   - un élément résistif agencé dans ledit passage interne de manière à créer une perte de charge du gaz traversant ledit passage,
   - un premier orifice de mesure traversant la paroi périphérique de l'élément de conduit entre l'orifice d'entrée et l'élément résistif,
   - et un deuxième orifice de mesure pratiqué dans la paroi périphérique de l'élément de conduit entre l'orifice de sortie et l'élément résistif,
- et le sous-ensemble de mesure comprend deux capteurs de pression dont les entrées de mesure sont respectivement connectées à des canaux de prise de pression en communication fluidique avec les premier et deuxième orifices de mesure lorsque le sous-ensemble de mesure est solidarisé à l'élément de conduit,
et dans lequel des membranes (24, 25 ; 31, 32) sont agencées au niveau des premier et deuxième orifices de mesure (14, 15) de l'élément de conduit (2) ou des canaux de prise de pression (31a, 32a) des capteurs de pression (33, 34),
**caractérisé en ce que** tout ou partie des membranes (24, 25 ; 31, 32) est en céramique et les membranes (24, 25 ; 31, 32) comprennent une multitude de pores dont la taille est inférieure à 100 nm.

Le fait d'utiliser des membranes en céramique confère au système différents avantages par rapport aux membranes classiques.

Ainsi, la matière céramique de chaque membrane, à savoir de la céramique de type oxyde d'aluminium, est extrêmement résistante et résiste donc facilement à des températures de l'ordre de 300°C, c'est-à-dire bien au-delà de la température de 134°C que l'on retrouve dans les cycles classiques de stérilisation à l'autoclave.

Par ailleurs, la matière céramique est chimiquement inerte, c'est-à-dire qu'elle ne s'altère pas au contact d'éléments chimiques, à savoir le gaz administré au patient, les produits de nettoyage..., sa biocompatibilité est satisfaite et sa durée de vie bien supérieure aux membranes actuellement connues.

De plus, en utilisant de la céramique, on peut obtenir des membranes de très faible taille de pores, à savoir une taille de pore de l'ordre de 100 nm (0,1µm) qui est largement suffisante pour garantir une parfaite protection des éléments placés derrière la membrane, lesquels éléments ne nécessitent alors aucun nettoyage particulier. En effet, la taille des micro-organismes pathogènes les plus petits, tels que les virus, susceptibles de se trouver dans les flux gazeux peut être de l'ordre d'à peine 0.2 µm, donc nettement supérieure à la taille des pores de la membrane en céramique selon l'invention. Ces micro-organismes pathogènes seront donc stoppés efficacement par les membranes en céramique utilisées dans le cadre de la présente invention.

Il est également utile de mentionner qu'une telle membrane peut être rendue hydrophobe par simple agencement des molécules d'oxyde d'aluminium les unes par rapport aux autres. Dans le cadre d'une utilisation proximale du système, il est en effet commun d'assister au phénomène de condensation de la vapeur d'eau, ce qui peut se traduire par la formation d'une pellicule d'eau. Le caractère hydrophobe de la membrane, en empêchant la formation d'une telle pellicule, évite que les pores ne se bouchent, ce qui sécurise l'utilisation du système.

Selon le cas, le système de l'invention peut comprendre l'une ou plusieurs des caractéristiques techniques suivantes :
- l'association des capteurs de pression est un capteur de pression différentielle à deux canaux de prise de pression.
- les membranes comprennent une multitude de pores dont la taille est inférieure ou égale à 90 nm, de préférence inférieure ou égale à 80 nm.
- les membranes comprennent une multitude de pores dont la taille des pores n'excède pas 70 nm.
- tout ou partie des membranes est en céramique hydrophobe.
- tout ou partie des membranes est en céramique de type oxyde d'aluminium.
- l'élément résistif est formé d'une pièce avec la paroi périphérique de l'élément de conduit.
- lorsque le sous-ensemble de mesure est solidarisé à l'élément de conduit, les entrées de mesure des capteurs de pression sont en communication fluidique, via les canaux de mesures, avec les premier et deuxième orifices de mesure de l'élément de conduit au niveau desquels sont agencées les membranes.
- le sous-ensemble de mesure comprend des liaisons électriques aptes à et conçues pour permettre un raccordement électrique des capteurs à un système de commande d'un ventilateur.
- le système de connexion/déconnexion permettant de solidariser mécaniquement le sous-ensemble de mesure à l'élément de conduit comprend une arête agencée sur l'élément de conduit et une fente agencée sur le sous-ensemble de mesure, ladite arête et ladite fente étant configurées et dimensionnées pour venir s'insérer l'une dans l'autre et à assurer ainsi la solidarisation du sous-ensemble de mesure à l'élément de conduit. En d'autres termes, la connexion du sous-ensemble de mesure à l'élément de conduit est assurée par une coopération de l'arête en saillie avec la fente.

L'invention concerne aussi un élément de conduit d'un système de mesure de pression selon l'invention comprenant une paroi périphérique tubulaire délimitant un passage interne de gaz reliant fluidiquement un orifice d'entrée à un orifice de sortie, un élément résistif agencé dans ledit passage interne de manière à créer une perte de charge du gaz traversant ledit passage, un premier orifice de mesure traversant la paroi périphérique de l'élément de conduit entre l'orifice d'entrée et l'élément résistif, et un deuxième orifice de mesure pratiqué dans la paroi périphérique de l'élément de conduit entre l'orifice de sortie et l'élément résistif, des membranes (24, 25 ; 31, 32) étant agencées au niveau des premier et deuxième orifices de mesure (14, 15), **caractérisé en ce que**
- tout ou partie des membranes (24, 25 ; 31, 32) est en céramique et
- les membranes (24, 25 ; 31, 32) comprennent une multitude de pores dont la taille est inférieure à 100 nm.

Selon le cas, l'élément de conduit de l'invention peut comprendre l'une ou plusieurs des caractéristiques techniques suivantes :
- les membranes comprennent une multitude de pores dont la taille des pores inférieure ou égale à 90 nm, de préférence inférieure ou égale à 80 nm.
- les membranes comprennent une multitude de pores dont la taille des pores n'excède pas 70 nm.
- les membranes sont en céramique de type oxyde d'aluminium.

Par ailleurs, l'invention porte aussi sur un sous-ensemble de mesure d'un système de mesure de pression selon l'invention comprenant deux capteurs de pression dont les entrées de mesure sont respectivement connectées à des canaux de prise de pression, **caractérisé en ce que** des membranes sont agencées dans les canaux de prise de pression.

Selon le cas, le sous-ensemble de mesure de l'invention peut comprendre l'une ou plusieurs des caractéristiques techniques suivantes :
- les membranes sont en céramique, de préférence une céramique hydrophobe.
- l'élément de mesure comprend, en outre, un capteur d'oxygène.
- l'élément de mesure comprend, en outre, un ou plusieurs éléments chauffant, de préférence une ou des résistances électriques.

En outre, l'invention concerne aussi une installation de ventilation d'un patient comprenant un ventilateur relié fluidiquement à un circuit patient, et un système de mesure de pression selon l'invention comprenant un élément de conduit selon l'invention, raccordé fluidiquement au circuit patient et un sous-ensemble de mesure selon l'invention relié électriquement audit ventilateur.

L'invention va maintenant être mieux comprise grâce à la description suivante faite en références aux Figures annexées parmi lesquelles :
- la FIG. 1 illustre un mode de réalisation d'un système de mesure selon la présente invention,
- la FIG. 2 illustre un mode de réalisation d'un élément de conduit selon la présente invention,
- les FIG. 3a à 3e illustre un mode de réalisation d'un sous-ensemble de mesure selon la présente invention, et
- la FIG. 4 schématise un mode de réalisation d'un système de mesure selon l'invention raccordé à une installation de ventilation d'un patient.

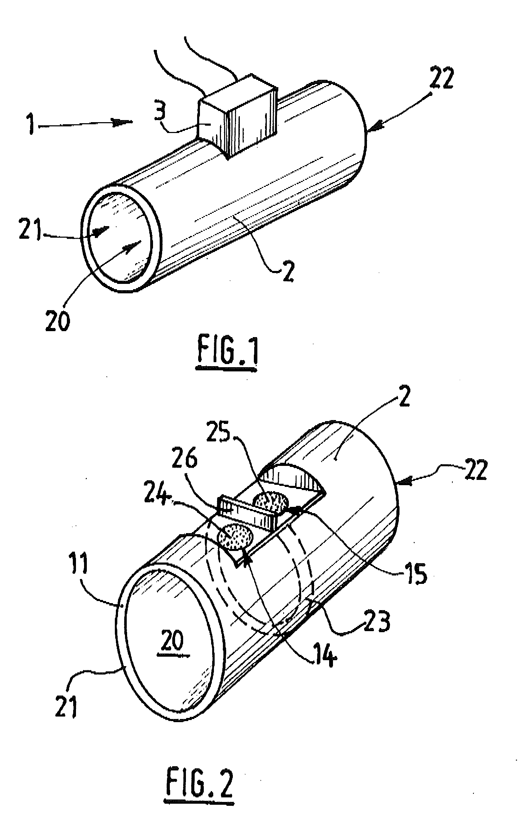

Comme schématisé en FIG. 1, un système de mesure 1 de pression (ou débit) apte à venir se raccorder au circuit patient d'un dispositif ventilateur ou analogue pour y effectuer des mesures de pression ou débit, comprend, selon un mode de réalisation, deux éléments principaux 2, 3 pouvant être connectés et déconnectés l'un de l'autre sans l'aide d'un quelconque outil, à savoir un élément de conduit 2 véhiculant le gaz et un sous-ensemble de mesure 3.

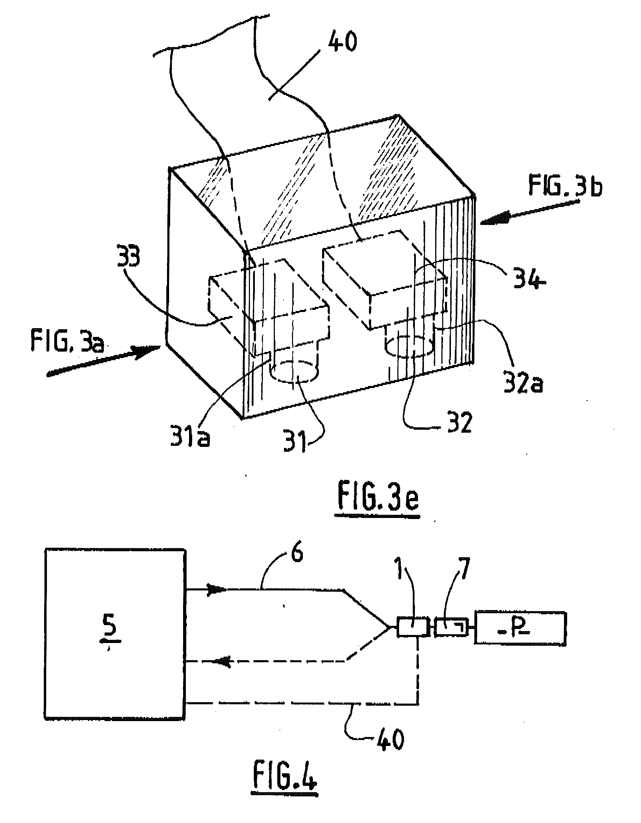

Plus précisément, comme montré en FIG. 2 et 4, l'élément de conduit 2 vient se raccorder directement sur le circuit patient 6 véhiculant le flux inspiré et expiré par le patient P, et habituellement au plus près de ce dernier pour permettre d'obtenir une mesure aussi précise que possible des pressions et débits gazeux, en particulier de ceux délivré par le ventilateur 5 relié fluidiquement au circuit 6 pour l'alimenter en gaz respiratoire qui est acheminé jusqu'au patient P.

L'élément de conduit 2 vient s'interconnecter de préférence entre l'extrémité d'un circuit patient 6, simple ou à double branche, et l'interface patient 7, tel qu'un tube endo-trachéal dans le cadre d'une ventilation invasice ou bien un masque lors d'une séance de ventilation non invasive. A cette fin, l'élément de conduit 2 est muni d'un orifice d'entrée 21 et un orifice de sortie 22 dimensionnés pour pouvoir venir se raccorder par emmanchement au circuit patient 6 ainsi qu'à l'interface 7 patient.

Au sein de l'élément de conduit 2, l'orifice d'entrée 21 est relié fluidiquement, via un passage interne 20 de gaz, à l'orifice de sortie 22. Par exemple, les diamètres des orifices d'entrée et de sortie 21 et 22 pourront être de 22 mm dans le cas d'une ventilation d'un adulte et de 15 mm en pédiatrie. Leurs dimensions sont préférentiellement choisies en fonction de la population de patients à traiter.

Dans tous les cas, l'élément de conduit 2 comprend en son centre, c'est-à-dire dans son passage interne 20, un élément résistif 23 comprenant un motif ou profil qui génère une perte de charge lorsqu'un débit de gaz vient le traverser.

L'élément résistif 23 agencé dans l'élément de conduit 2 peut être soit un élément rapporté fixé, par exemple collé, sur la paroi interne de l'élément de conduit 2, soit être formé d'une seule pièce avec le corps de l'élément de conduit 2, par exemple par moulage. Avantageusement, l'élément résistif 23 est formé d'une partie de la paroi interne de l'élément de conduit 2 se projetant radialement vers l'intérieur, c'est-à-dire la lumière du passage interne 20 de gaz traversant le conduit 2. La paroi intérieure de l'élément de conduit 2 portant élément résistif 23 peut par exemple avoir une forme de tuyère de Laval, c'est-à-dire une forme générale convergente/divergente.

Dans tous les cas, la perte de charge s'exprime, en fonction du sens d'écoulement du gaz, comme la différence entre la pression en amont de l'élément résistif 23 et la pression gazeuse en aval de celui-ci.

Pour permettre de prélever du gaz sous pression en amont et en aval de l'élément résistif 23, l'élément de conduit 2 comprend :
- un premier orifice de mesure 14 pratiqué dans la paroi périphérique du conduit 2 entre l'orifice d'entrée 21 et l'élément résistif 23 de manière à pouvoir opérer une prise de pression gazeuse dans l'élément de conduit 2 avant toute perte de charge engendrée par l'élément résistif 23,
- et un deuxième orifice de mesure 15 pratiqué, quant à lui, dans la paroi périphérique du conduit 2 entre l'orifice de sortie 22 et l'élément résistif 23, de manière à pouvoir opérer une prise de pression gazeuse dans l'élément de conduit 2 après la perte de charge engendrée par l'élément résistif 23.

Ces pressions sont donc mesurées (i.e. transmission du signal par effet de compression décompression des gaz) en amont et en aval de l'élément résistif 23 et se transmettent alors au capteur 33, par exemple un capteur différentiel comme expliqué ci-après, en passant à travers deux membranes poreuses 24 et 25 agencées au niveau des premier et deuxième orifices de mesure 14, 15, respectivement.

En d'autres termes, les membranes poreuses 24, 25 comprennent une multitude de pores dont la taille n'excède pas 70 nm, les rendant ainsi imperméables à tout agent bactérien et protégeant alors le sous-ensemble de mesure 3 de toute contamination. En fait, les agents bactériens sont généralement de taille nettement supérieure à 100 nm, typiquement leur taille oscille entre environ 200 et 3000 nm.

Dans tous les cas, placer une membrane poreuse 24, 25 entre le conduit transmettant le gaz au patient, potentiellement contaminé, et le capteur 33 réalisant la mesure empêche toute contamination dudit capteur 33.

L'épaisseur des membranes 24 et 25 est comprise entre 10 et 300 µm, préférentiellement entre 25 et 150 µm, par exemple de l'ordre de 50 µm, afin de leur conférer une résistance mécanique suffisante aux vibrations et chocs auxquels elles pourraient être soumises.

A titre d'exemple, on peut utiliser en tant que membranes 24, 25, celles commercialisées par Synkera Technologies sous la référence SM-73-50-10x10.

Avantageusement, on utilise des membranes 24, 25 en céramique, ce qui les rend très résistantes et donc aptes à supporter de multiples cycles de désinfection/stérilisation, c'est-à-dire des passages à l'autoclave à 134°C pendant plusieurs minutes à chaque fois.

Par ailleurs, le matériau constituant l'élément de conduit 2 est du plastique résistant aux cycles de désinfection/stérilisation de manière à pouvoir réutiliser le conduit 2 après nettoyage ou alors un matériau moins résistant mais plus économique, tel par exemple le polycarbonate, de manière à obtenir un conduit à usage unique, c'est-à-dire qui est remplacé à chaque nouveau patient.

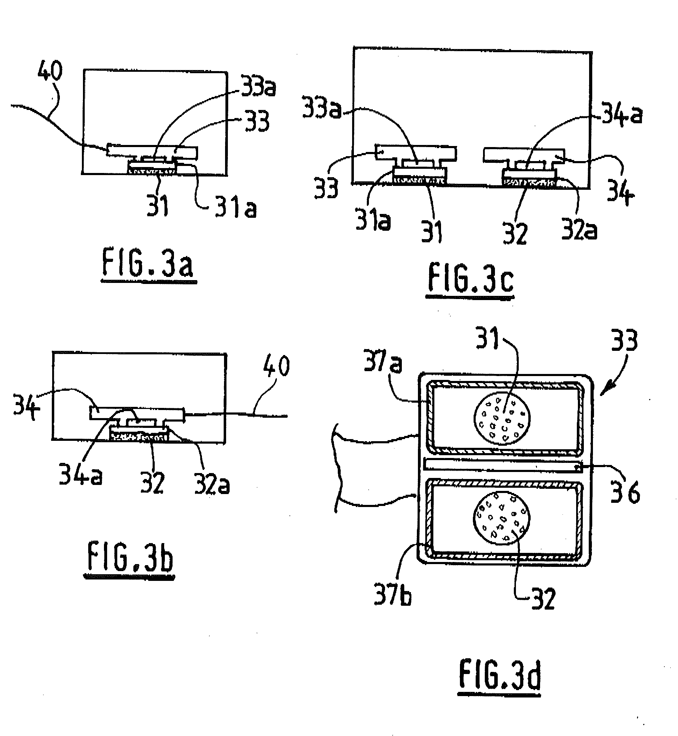

Les FIG. 3a à 3e illustrent l'architecture du sous-ensemble de mesure 3 servant à réaliser la mesure du différentiel de pression créé par le flux d'air traversant l'élément résistif 23 agencé dans l'élément de conduit 2.

Avantageusement, le sous-ensemble de mesure 3 peut comprendre lui aussi deux autres membranes poreuses 31, 32 agencées dans les canaux de prise de pression 31a, 32a du capteur 33 et qui permettent de protéger les parties internes du sous-ensemble de mesure 3, c'est-à-dire du capteur 33, de toute pollution lorsque, par exemple, le sous-ensemble de mesure 3 est déconnecté de l'élément de conduit 2 et laissé à l'air libre en attente d'une nouvelle connexion. La taille des pores de ces membranes sera ainsi largement supérieure à celles présentes sur les membranes 24 et 25, par exemple de l'ordre de 500µm.

Dans ce cas, lorsque l'élément 3 est connecté de manière étanche à l'élément de conduit 2, comme illustré en FIG 1, la membrane 31 est directement en contact avec la membrane 24 et, de la même manière, la membrane 32 est en contact avec la membrane 25. Les pressions gazeuses prélevées en amont et en aval de l'élément résistif 23 en présence d'un flux d'air se transmettent alors à l'élément de mesure 3 à travers les membranes 24 et 31, d'une part, et 25 et 32, d'autre part.

Le système de connexion/déconnexion permettant de connecter l'élément 3 de manière étanche à l'élément de conduit 2 met en jeu 2 formes complémentaires. Ainsi, l'élément de conduit 2 dispose d'une arête ou paroi en saillie 26 conformée et dimensionnée pour parfaitement s'insérer dans une fente 36 de l'élément 3. Cette liaison entre arête 26 et fente 36 assure ainsi le positionnement et le maintien de l'élément 3 sur le conduit 2.

Afin de réaliser l'étanchéité entre les membranes 24 et 31, d'une part, et 25 et 32, d'autre part, l'élément de mesure 33 dispose également de 2 joints 37a et 37b qui entourent les membranes susmentionnées et viennent alors en contact avec les surfaces planes du conduit 2 de part et d'autre de l'arête 26.

Intégré dans l'élément de mesure 3, un capteur de pression 33 utilisant une cellule piezorésistive 33a (par exemple All Sensors DIE-L30G) réalise une mesure de pression en amont de la surface résistive 23 par le biais du canal de mesure 31a, en contact avec la membrane 31.

Par une disposition similaire, un capteur de pression 34 utilisant la même cellule piezorésistive 34a (par exemple All Sensors DIE-L30G) réalise une mesure de pression en aval de la surface résistive 23 par le biais du canal de mesure 32a, en contact avec la membrane 32. Le positionnement de ce capteur 34 permet également d'obtenir la pression appliquée aux voies aériennes du patient.

Les capteurs 33 et 34 sont alimentés par une liaison électrique 40, connectée par exemple à un ventilateur 5, comme schématisé en FIG. 4, et à son système de commande, qui comprend typiquement une carte à microprocesseur, et renvoient également à travers cette liaison électrique 40 une image de la pression mesurée en amont et en aval de l'élément résistif 23.

La carte de commande du ventilateur peut alors exploiter ces informations pour en déduire :
- la pression qui règne au niveau des voies aériennes du patient via une exploitation du signal issu du capteur 34a, et/ou
- le débit qui traverse l'élément 2 à l'instant de la mesure par le biais de la pression différentielle de part et d'autre de la surface 23, via une soustraction des signaux issus des capteurs 33 et 34.

On veillera à minimiser le volume qui existe entre la membrane 24 et le capteur 33 ainsi que la membrane 25 et le capteur 34. Ces membranes, constituées de pores de très faible diamètre sont en effet des éléments résistifs qui peuvent nuire au temps de réponse des capteurs de pression. A ce titre, une réalisation des pièces constitutives de l'ensemble 3 par micro usinage voire micro moulage est préférée.

Dans tous les cas, le temps de réponse de deux éléments de mesure 33 et 34 ne dépassera quelques ms.

Le système de mesure 1 de l'invention permet ainsi de mesurer par le biais de l'élément de mesure 3, tout débit traversant l'élément de conduit 2, ainsi que la pression gazeuse des voies aériennes du patient.

Afin de s'affranchir des problèmes liés à l'humidité contenue dans les gaz inspirés par le patient pouvant être préalablement humidifiés et dans les gaz expirés par le patient, l'élément de mesure 3 est pourvu de moyens permettant d'éviter ou minimiser toute condensation d'eau.

En effet, si l'eau à l'état de vapeur n'est pas problématique pour le système de mesure 1, il apparaît que sa condensation peut non seulement obstruer les pores des membranes 24, 25 mais aussi endommager les capteurs de pression 33, 34.

De là, pour empêcher toute condensation d'eau, on agence au niveau de l'élément de mesure 3, un ou plusieurs éléments chauffant, telles des résistances électriques, de manière à ce que la température à l'intérieur de l'enveloppe de l'élément de mesure 3 et jusqu'aux membranes 31, 32, avoisine les 40°C.

Par diffusion thermique, les membranes 24, 25 seront alors elles-mêmes portées à une température supérieure à celle des gaz inspirés et expirés, limitant alors la condensation de ces gaz à la surface de ces membranes 24, 25 ainsi que dans l'élément de mesure 3.

Par ailleurs, on choisit préférentiellement des membranes 24, 25 en céramique hydrophobes afin d'éviter que d'éventuelles projections d'eau ne viennent obstruer leurs pores.

Selon un autre aspect, l'élément de mesure 3 peut aussi intégrer un capteur d'oxygène, par exemple le capteur d'O₂ de type SMSI commercialisé par la société S4MS, afin de permettre une mesure proximale de la fraction inspirée (FiO₂) puis expirée (FeO₂) par le patient.

## Revendications

1. Système de mesure de pression (1) comprenant un élément de conduit (2), un sous-ensemble de mesure (3) et un système de connexion/déconnexion permettant de solidariser mécaniquement le sous-ensemble de mesure (3) à l'élément de conduit (2) ou, à l'inverse, de les désolidariser l'un de l'autre, et dans lequel :
- l'élément de conduit (2) comprend :
. une paroi périphérique (11) tubulaire délimitant un passage interne (20) de gaz reliant fluidiquement un orifice d'entrée (21) à un orifice de sortie (22),
. un élément résistif (23) agencé dans ledit passage interne (20) de manière à créer une perte de charge du gaz traversant ledit passage (20),
. un premier orifice de mesure (14) traversant la paroi périphérique (11) de l'élément de conduit (2) entre l'orifice d'entrée (21) et l'élément résistif (23),
. et un deuxième orifice de mesure (15) pratiqué dans la paroi périphérique (11) de l'élément de conduit (2) entre l'orifice de sortie (22) et l'élément résistif (23),
- et le sous-ensemble de mesure (3) comprend deux capteurs de pression (33, 34) dont les entrées de mesure sont respectivement connectées à des canaux de prise de pression (31a, 32a) en communication fluidique avec les premier et deuxième orifices de mesure (14, 15) lorsque le sous-ensemble de mesure (3) est solidarisé à l'élément de conduit (2),
et dans lequel des membranes (24, 25 ; 31, 32) sont agencées au niveau des premier et deuxième orifices de mesure (14, 15) de l'élément de conduit (2) ou des canaux de prise de pression (31a, 32a) des capteurs de pression (33, 34),
**caractérisé en ce que** :
- tout ou partie des membranes (24, 25 ; 31, 32) est en céramique et
- les membranes (24, 25 ; 31, 32) comprennent une multitude de pores dont la taille est inférieure à 100 nm.

2. Système de mesure de pression selon la revendication précédente, **caractérisé en ce que** l'association des capteurs de pression (33, 34) est un capteur de pression différentielle à deux canaux de prise de pression (31a, 32a).

3. Système de mesure de pression selon l'une des revendications précédentes, **caractérisé en ce que** les membranes (24, 25 ; 31, 32) comprennent une multitude de pores dont la taille est inférieure à 90 nm, de préférence la taille des pores n'excède pas 70 nm.

4. Système de mesure de pression selon l'une des revendications précédentes, **caractérisé en ce que** tout ou partie des membranes (24, 25 ; 31, 32) est en céramique hydrophobe et/ou en céramique de type oxyde d'aluminium.

5. Système de mesure de pression selon l'une des revendications précédentes, **caractérisé en ce que** l'élément résistif (23) est formé d'une pièce avec la paroi périphérique (11) de l'élément de conduit (2).

6. Système de mesure de pression selon l'une des revendications précédentes, **caractérisé en ce que**, lorsque le sous-ensemble de mesure (3) est solidarisé à l'élément de conduit (2), les entrées de mesure des capteurs de pression (33, 34) sont en communication fluidique, via les canaux de mesures (31a, 32a), avec les premier et deuxième orifices de mesure (14, 15) de l'élément de conduit (2) au niveau desquels sont agencées les membranes (24, 25).

7. Système de mesure de pression selon l'une des revendications précédentes, **caractérisé en ce que** le sous-ensemble de mesure (3) comprend des liaisons électriques (40) aptes à et conçues pour permettre un raccordement électrique des capteurs (33) et (34) à un système de commande d'un ventilateur.

8. Système de mesure de pression selon l'une des revendications précédentes, **caractérisé en ce que** le système de connexion/déconnexion permettant de solidariser mécaniquement le sous-ensemble de mesure (3) à l'élément de conduit (2) comprend une arête (26) agencée sur l'élément de conduit (2) et une fente (36) agencée sur le sous-ensemble de mesure (3), ladite arête (26) et ladite fente (36) étant configurées et dimensionnées pour venir s'insérer l'une dans l'autre et à assurer ainsi la solidarisation du sous-ensemble de mesure (3) à l'élément de conduit (2).

9. Elément de conduit (2) d'un système de mesure de pression selon l'une des revendications précédentes comprenant une paroi périphérique (11) tubulaire délimitant un passage interne (20) de gaz reliant fluidiquement un orifice d'entrée (21) à un orifice de sortie (22), un élément résistif (23) agencé dans ledit passage interne (20) de manière à créer une perte de charge du gaz traversant ledit passage (20), un premier orifice de mesure (14) traversant la paroi périphérique (11) de l'élément de conduit (2) entre l'orifice d'entrée (21) et l'élément résistif (23), et un deuxième orifice de mesure (15) pratiqué dans la paroi périphérique (11) de l'élément de conduit (2) entre l'orifice de sortie (22) et l'élément résistif (23), des membranes (24, 25 ; 31, 32) étant agencées au niveau des premier et deuxième orifices de mesure (14, 15), **caractérisé en ce que**
- tout ou partie des membranes (24, 25 ; 31, 32) est en céramique et
- les membranes (24, 25 ; 31, 32) comprennent une multitude de pores dont la taille est inférieure à 100 nm.

10. Elément de conduit (2) selon la revendication 9, **caractérisé en ce que** les membranes (24, 25) comprennent une multitude de pores dont la taille est inférieure à 90 nm, de préférence la taille de pores n'excède pas 70 nm.

11. Elément de conduit (2) selon l'une des revendications 9 ou 10, **caractérisé en ce que** les membranes (24, 25) sont en céramique de type oxyde d'aluminium.

12. Sous-ensemble de mesure (3) d'un système de mesure de pression selon l'une des revendications 1 à 8 comprenant deux capteurs de pression (33, 34) dont les entrées de mesure sont respectivement connectées à des canaux de prise de pression (31a, 32a), **caractérisé en ce que** des membranes (31, 32) sont agencées dans les canaux de prise de pression (31a, 32a).

13. Sous-ensemble de mesure (3) selon la revendication 12, **caractérisé en ce qu'**il comprend, en outre, un capteur d'oxygène.

14. Sous-ensemble de mesure (3) selon l'une des revendications 12 ou 13, **caractérisé en ce qu'**il comprend, en outre, un ou plusieurs éléments chauffant, de préférence une ou des résistances électriques.

15. Installation de ventilation d'un patient comprenant :
- un ventilateur (5) relié fluidiquement à un circuit patient (6), et
- un système de mesure de pression (1) selon l'une des revendications 1 à 8 comprenant :
o un élément de conduit (2) selon l'une des revendications 9 à 11, raccordé fluidiquement au circuit patient (6) et
o un sous-ensemble de mesure (3) selon l'une des revendications 12 à 14 relié électriquement (40) audit ventilateur (5).
